# EUROPEAN PATENT APPLICATION

(11) **EP 4 803 128 A1**
(43) Date of publication of application: **09.09.2026**
(21) Application number: 26162797.0
(22) Date of filing: 06.03.2026
(51) Int. Cl.: A61M 25/02

(54) **SUTURE PAD AND SHEATH INSERT**

(30) Priority: 07.03.2025 US 202563768416 P
(71) Applicant: Abiomed, Inc., Danvers, MA 01923 (US)
(72) Inventor: SEGURA ORTEGA, Carlos Alejandro, Danvers, 01923 (US); JAHANGIR, Emilia, Danvers, 01923 (US); FINCK, Adam, Danvers, 01923 (US); NORRAY, Kristen, Danvers, 01923 (US)
(74) Representative: Wittmer, Maximilian

(57) **Abstract**

A suture pad and sheath insert, and a system using such inserts, may be provided. The insert may include a central body having an outer surface, a first end and a second end. The central body may define a lumen extending through the central body. The insert may include two wings, each wing extending radially outward from the outer surface of the central body. The two wings may be configured such that the insert is symmetrical around a first plane that may be parallel to and passing through the central axis, where the first plane may pass between the first surface and second surface of each wing and may be symmetrical around a second plane that may be parallel to and passing through the central axis, where the second plane may be perpendicular to the first plane.

## Description

### CROSS-REFERENCE TO RELATED APPLICATIONS

The present application claims priority to U.S. Provisional Patent Application No. 63/768,416, filed March 7, 2025, the contents of which are incorporated by reference herein in its entirety.

### TECHNICAL FIELD

The present disclosure is drawn to suture pad and sheath inserts, and specifically to inserts that can provide fixation and hemostasis features.

### BACKGROUND

Hemostasis is a factor physicians consider when using certain medical device products, such as catheter-based blood pumps). In some current approaches, sutures may be used to achieve that hemostasis.

### BRIEF SUMMARY

In various aspects, a suture pad and sheath insert may be provided. The insert may include a central body having an outer surface, a first end and a second end. The central body may define a lumen extending from the first end to the second end, where the lumen may define a central axis of the suture pad and sheath insert. The insert may include two wings, each wing extending radially outward from the outer surface of the central body. Each wing may define a plurality of openings, each of which may extend from a first surface of the wing to a second surface of the wing opposite the first surface. The two wings may be configured such that the suture pad and sheath insert is symmetrical around a first plane that may be parallel to and passing through the central axis, where the first plane may pass between the first surface and second surface of each wing. The two wings may be configured such that the suture pad and sheath insert may be symmetrical around a second plane that may be parallel to and passing through the central axis, where the second plane may be perpendicular to the first plane. The two wings may be configured such that the wings may be symmetrical around a third plane that may be orthogonal to the central axis.

An axial length of the lumen may be the same as minimum axial distance between a first end of the wing and a second end of the wing. An axial length of the lumen may be greater than a minimum axial distance between a first end of the wing and a second end of the wing.

An axial length from the first end of the central body to a first end of the wing may be the same as the axial length from the second end of the central body to a second end of the wing. An axial length from the first end of the central body to a first end of the wing may be greater than the axial length from the second end of the central body to a second end of the wing. An axial length from the first end of the central body to a first end of the wing may be less than the axial length from the second end of the central body to a second end of the wing.

Each wing may have a minimum axial length between a first end of the wing and a second end of the wing, and a maximum axial length between a first end of the wing and a second end of the wing, where the minimum axial length may be different from the maximum axial length. Each wing may be configured such that a minimum axial length exists at the point where the wing contacts the central body.

The outer surface of the central body at the first end of the central body may be tapered, such that an outer diameter at the first end is smaller than an outer diameter at a different portion of the central body.

In various aspects, a system may be provided. The system may include a suture pad and sheath insert as disclosed herein, and at least one tubular member disposed at least partially though the lumen, an inner surface of the lumen forming an interference fit with the at least one tubular member. A hub maybe operably coupled to an end of the at least one tubular member. A hub maybe operably coupled to the second end of the lumen. The hub may comprise a hemostasis valve. The hub may comprise a side port, and a secondary tubular member may be coupled to the side port. The at least one tubular member may be a repositioning sheath extending through the lumen.

### BRIEF DESCRIPTION OF FIGURES

The accompanying drawings, which are incorporated in and constitute a part of this specification, illustrate embodiments of the present invention and, together with a general description of the invention given above, and the detailed description of the embodiments given below, serve to explain the principles of the present invention.
Figure 1A is an illustration showing an isometric projection of a disclosed suture pad.
Figure 1B is an illustration showing a top view of the suture pad of Figure 1A.
Figure 1C is an illustration showing a side view of the suture pad of Figure 1A.
Figures 1D-1F are illustrations showing top views of the
Figure 2 is an illustration showing an isometric projection of another disclosed suture pad.
Figure 3 is an illustration showing an isometric projection of a disclosed sheath insert.
Figure 4 is an illustration showing an exploded view of a suture pad and a sheath insert.
Figure 5 is an illustration of a system utilizing a disclosed insert.
Figure 6 is an illustration of a system utilizing a disclosed insert.

It should be understood that the appended drawings are not necessarily to scale, presenting a somewhat simplified representation of various features illustrative of the basic principles of the invention. The specific design features of the sequence of operations as disclosed herein, including, for example, specific dimensions, orientations, locations, and shapes of various illustrated components, will be determined in part by the particular intended application and use environment. Certain features of the illustrated embodiments have been enlarged or distorted relative to others to facilitate visualization and clear understanding. In particular, thin features may be thickened, for example, for clarity or illustration.

### DETAILED DESCRIPTION

As is known, hemostasis is an important consideration during and after medical procedures, such as procedures including catheter-based blood pumps. In some embodiments, challenges with achieving hemostasis may deter adoption of certain medical procedures. The inventors have recognized that advantages may be achieved by the suture pad and/or sheath insert described herein. For example, in some embodiments, the disclosed suture pad and/or insert may improve the ease with which hemostasis and longer-term stability can be achieved (e.g., such as once a patient leaves the catheterization lab or operating room. As will be described herein, in some embodiments, the suture pad and insert may be separate components operably coupled together, may be attached, and/or may be integrally formed.

In some embodiments, a suture pad and sheath insert may be provided. As disclosed herein, in some embodiments, the sheath and/or suture pad may be used to insert a medical device into a patient (e.g., by passing the medical device through the sheath and/or suture pad), and to allow the suture pad to be attached to a skin of the patient.

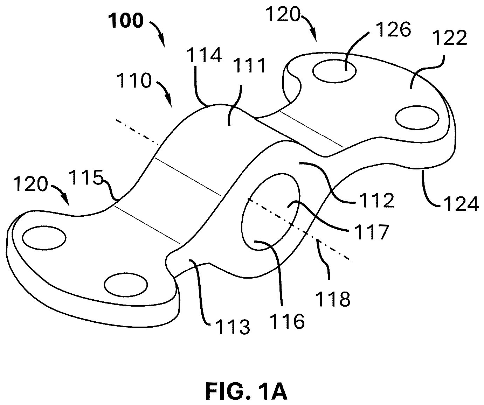

Referring to FIG. 1A, the suture pad **100** may include a central body **110** and a pair of wings **120.** The central body and wings may form an integral part. The central body may have an outer surface **111,** a first end **112** (*e.g*., a front side) and a second end **114** (*e.g*., a back side). The central body may define a lumen **116** extending from the first end to the second end. The lumen may be formed by an inner surface **117** of the central body, and the lumen may define a central axis **118** of the suture pad and sheath insert. The lumen may have an predetermined inner diameter **130.**

As seen, each wing may extend radially outward from the outer surface of the central body. Each wing may be defined by a first surface **122** (e.g., a top surface) and second surface **124** opposite the first surface (e.g., a bottom surface). Each wing may include a plurality of openings **126,** each of which may extend from the first surface of the wing to the second surface of the wing.

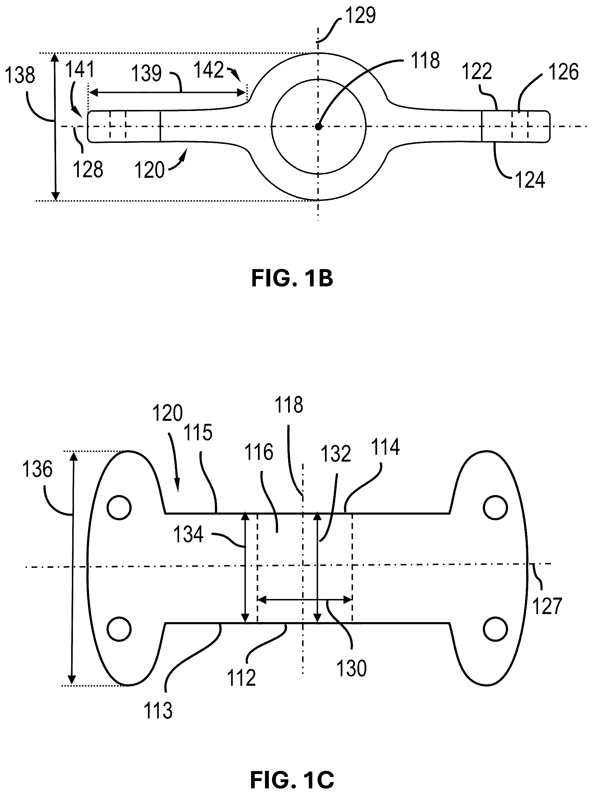

Each wing may be center aligned (e.g., aligned along a central axis or first plane **128),** allowing for ideal placement regardless of orientation. That is, each wing may mirror the other, on opposite sides of the central axis. The wings may have multiple degrees of symmetry. For example, referring to FIG. 1B, a top view of the same insert shown in FIG. 1A can be seen. As shown, the two wings may be configured such that the insert may be symmetrical around a first plane **128** that may be parallel to and passing through the central axis **118,** where the first plane may pass between the first surface **122** and second surface **124** of each wing. The two wings may be configured such that the insert may be symmetrical around a second plane **129** that may be parallel to and passing through the central axis, where the second plane **129** may be perpendicular to the first plane **128.** Referring to FIG. 1C, the wings may be configured such that the wings may be symmetrical around a third plane **127,** where the third plane may be orthogonal to the central axis **118.** As will be appreciated, in such embodiments, the configuration of the suture pad may provide a physician with more flexibility in positioning the sheath insert against a paint for adhering a medical device to the patient (e.g., such as during and/or after a medical procedure, when hemostasis needs to be maintained.

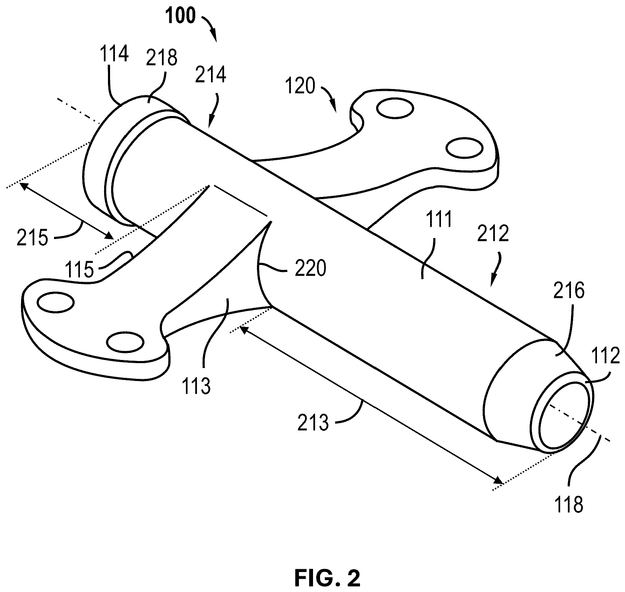

Axial lengths of the central body and the wings may vary independently. Referring to FIG. 1C, each wing may, independently, have a minimum axial length **134** and a maximum axial length **136** between a first end **113** (*e.g.*, a bottom end) and a second end **115** (*e.g.,* a top end) of the wing. The minimum axial length may be the same (or may be different) from the maximum axial length. In some embodiments, each wing may be configured such that a minimum axial length exists at the point where the wing contacts the central body (see, e.g., reference **220** in FIG. 2).

The central body may have a maximum outer diameter **138** (this may be the thickest portion of the suture pad, and is preferably a distance orthogonal to first plane **128).** Each wing may have a wing length **139** from the central body **110** to a wing end (such as second end **141)** (*e.g.*, in a direction perpendicular to the central axis **118).** The wing length may vary. For example, in some embodiments, the wing length may be no more than 20 cm, no more than 15 cm, no more than 12.5 cm, no more than 10 cm, or no more than 7.5 cm. In some preferred embodiments, the length may be no more than 5.25 cm, or no more than 2.75 cm. The wing length for one wing may be the same, or different, from the wing length of an opposing wing.

In some embodiments, as shown in FIG. 1B for example, each wing may have a wing length **139** (sometimes referred to as a radial length, as it is in a direction orthogonal to the axis of the suture pad) extending from a first end **142** attached at the central body to a second end **141** having the plurality of openings **126** for attaching the suture pad to a patient. In some embodiments, the radial length of each wing may be up to 1 inch (2.54 cm) long. For example, each wing may be between 0.5 and 1 inches (1.27 cm to 2.54 cm) long. As will be appreciated, each wing may be the same length, although a first wing may have a different length than the second wing.

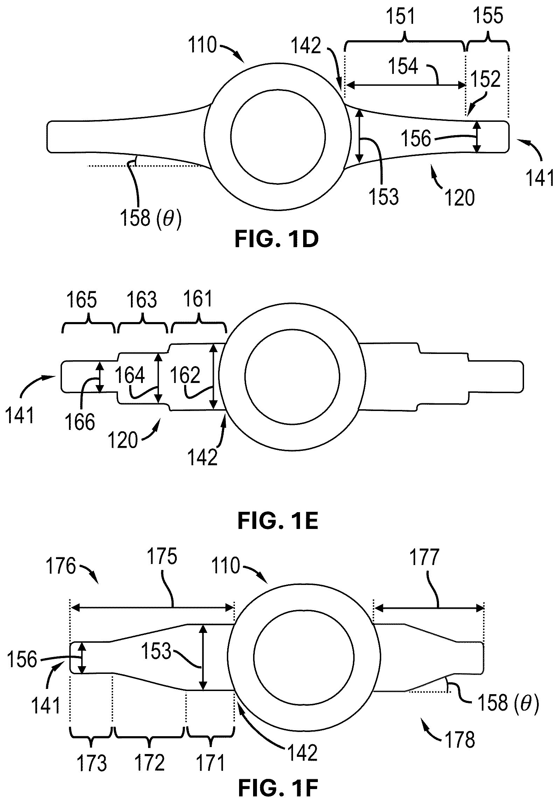

In some embodiments, each wing may have a constant thickness (e.g., the length at or near the second end with the plurality of openings). In other embodiments, each wing may have a thickness that varies between the first end **142** and the second end **141.** For example, a thickness T of the wing may decrease in a tapered fashion from the first end towards the second end. In some embodiments, the thickness T may taper from the first end, the entire length of the wing, to the second end. In other embodiments, as shown in FIG. 1D, the wing thickness may taper from the first end **142** to a position **152** in between the first end **142** and the second end **141.** For example, the wing may taper 50% of the length of the wing (e.g., from the first end **142** to a portion halfway between the first and second ends). The wing may also taper 75% of the length of the wing (e.g., from the first end to a portion 75% of the length of the wing). As will be appreciated, in such embodiments, the other portion of the wing may have a uniform thickness, although it may have other suitable arrangements. Thus, in some embodiments, the wing may have a tapering portion **151** that may have a first thickness **153** at the first end **142,** and which tapers down to a second thickness **156** at a position **152** intermediate the first end and second end. The position **152** may be a distance **154** from the second end that may be, e.g., 5% - 95% the total length of the wing (e.g., wing length **139),** and preferably 50%-75% the total length of the wing. The wing may then have a non-tapering portion **155** that has a uniform thickness from that intermediate position (*e.g.*, position **152)** to the second end **141** of the wing.

In some embodiments, there may be no tapering (i.e., a taper angle of 0). In some embodiments, the taper angle *θ* **158** at any point of the taper may be greater than 0 degrees and no more than 75 degrees. In some embodiments, the taper angle may preferably be greater than 0 degrees and less than 50 degrees, and more preferably less than 45 degrees. In some embodiments, the taper may have a constant taper angle. In some embodiments, the taper angle may vary over the length of the taper.

Referring to FIG. 1E, in still other embodiments, the thickness of the wing may decrease in a stepped fashion from the first end toward the second end. For example, a first portion **161** may include a first thickness **162** and a second portion **163** may have a second thickness **164.** The wing also may have a third portion **165** with a third thickness **166.** As will be appreciated in such embodiments, the first thickness may be different from at least the second thickness.

Various combinations of tapering, step changes in thickness, and non-tapering are envisioned. In one example, shown in FIG. 1F, as you move from a first end **142** to a second end **141** of the wing, a first portion **171** may be non-tapering with a first thickness **153,** a second portion **172** may taper at a constant taper angle from the first thickness **153** to a second thickness **156,** and a third portion **173** may be non-tapering with the second thickness **(156).**

Referring to FIG. 1C, in some embodiments, an axial distance (e.g., axial length **132)** between the first end and the second end of the central body (*e.g*., a length of the lumen) may be the same as minimum axial length **134** between a first end of the wing and a second end of the wing. In some embodiments, an axial length **132** of the lumen may be greater than the minimum axial length **134** of the wings. In some embodiments, an axial length **132** of the lumen may be greater than the maximum axial length **136** of the wings.

As will be appreciated, although the wings of the suture pad are often shown having multiple degrees of symmetry, in other embodiments, the first wing may differ from the second wing. For example, referring to FIG. 1F, a length **175** (*e.g.*, from the first end **142** to the second end **141)** of the first wing **176** may be different than the length **177** of the second wing **178.** In some embodiments, the taper angle *θ* **158** of the first wing **176** may be different from the taper angle of the second wing **178.**

In some embodiments, the central body and wings may be made of a polymeric material, and may be a pliable, resilient, flexible polymeric material. In some embodiments, the polymeric material may be a thermoplastic elastomer (TPE). Non-limiting examples may include VERSAFLEX^{®} TPE alloys made by GLS Corporation.

Referring to FIG. 2, an embodiment of a suture pad with a longer central body can be seen. The positioning of the wings relative to the central body may vary, such that there may be a first end portion **212** extending from the wings to the first end, and a second end portion **214** extending from the wings to the second end. In some embodiments, an axial length **213** of the first end portion (e.g., from the first end **112** of the central body to a first end **113** of the wing) may be the same as the axial length **215** of the second end portion (e.g., from the second end **114** of the central body to a second end **115** of the wing). In some embodiments, the axial length **213** of the first end portion may be greater than the axial length **215** of the second end portion. In some embodiments, an axial length **213** of the first end portion may be less than the axial length **215** of the second end portion.

The outer surface **111** of the central body at the first end **112** of the central body may have a taper **216,** such that an outer diameter at the first end is smaller than an outer diameter at a different portion of the central body, such as at the second end **114.**

The second end may include a protrusion **218** (or a depression).

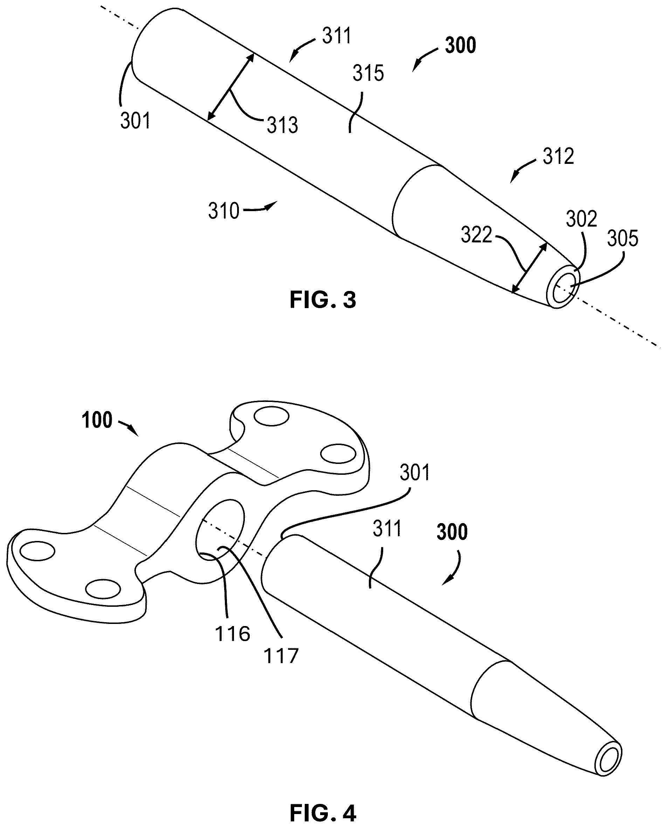

Referring to FIG. 3, a sheath insert **300** may include a sheath body **310** having a proximal end **301** and a distal end **302,** with a lumen **305** extending therethrough. The sheath body may consist of a cylindrical body. The sheath insert may have a constant outer diameter, or may have an outer diameter that varies in an axial direction. For example, as shown in FIG. 3, a sheath insert may have a proximal portion **311** with a first outer diameter (e.g., outer diameter **313)** that is constant, and a distal portion **312,** where the outer diameter **322** reduces (e.g., tapers) towards the distal end **302,** which has the smallest outer diameter.

Referring to FIG. 4, an outer surface **315** of the sheath insert is intended to be received by an inner surface **117** of lumen **116** of the suture pad **100.** The sheath insert may be permanently coupled to the suture pad. For example, the sheath insert may be glued into the lumen, or screws may be inserted to prevent movement of the sheath insert relative to the suture pad.

To introduce a blood pump to a patient, an introducer sheath may first be utilized. Such a sheath often may have a relatively large diameter (e.g., 23 Fr introducers are relatively common). Once the pump is introduced, through the introducer sheath, the introducer sheath can be peeled away, and a smaller repositioning sheath (e.g., 11 Fr inner diameter, 15 Fr outer diameter are commonly used) can be put in its place. In some embodiments, the venotomy hole left by the introducer sheath may be substantially larger than the outer diameter of the repositioning sheath (e.g., a 23 Fr introducer may leave a 27 Fr venotomy hole), leaving a sizeable gap (e.g., using the diameters listed here, the gap would be on the order of 12 Fr). In such embodiments, the sheath insert (for the repositioning sheath) may be sized so as to close this gap and achieve hemostasis by plugging the access site hole as soon as the repositioning sheath is advanced into the patient. The suture pad may then provide the means of fixating the sheath to the patient so that hemostasis is stable.

Reaching stable hemostasis, accounting for catheter movement and manipulation (e.g., a characteristic of ambulation), is extremely important for patient comfort and users' (e.g., all staff involved with patient care) peace of mind. The disclosed devices and systems reliably achieve hemostasis by simple anchoring to skin via the suture pad.

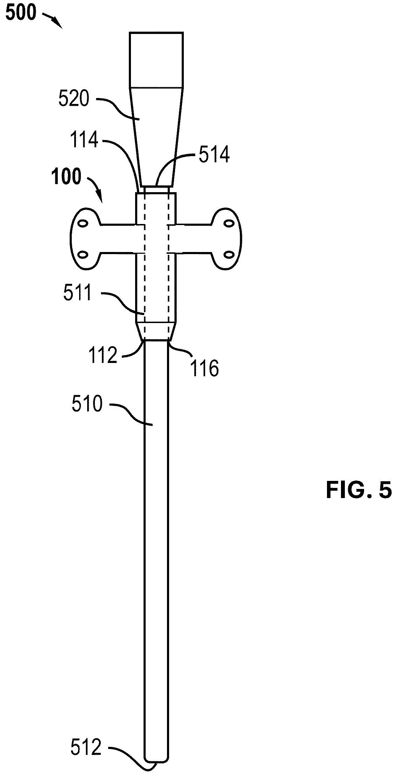

In various aspects, a system may be provided. Referring to FIG. 5, a system **500** may include a suture pad **100** and sheath insert **300** as disclosed herein. The system may include at least one tubular member **510** disposed at least partially though the lumen **116** (here, a portion **511** of the tubular member extends through the lumen **116),** an inner surface of the lumen forming an interference fit with the at least one tubular member.

The tubular member may be, *e.g.*, a repositioning sheath extending through the lumen. The tubular member may be a cannula extending only partially through the lumen.

In some embodiments, tubular member may be coupled to the insert only via the interference fit. In some embodiments, the coupling of the tubular member and the insert may be free of a glue. The tubular member may include a lumen extending from a distal end **512** to a proximal end **514.**

In some embodiments, a hub **520** may be operably coupled to the proximal end **514** of the tubular member. In some embodiments, the hub may be connected to the proximal end of the tubular member.

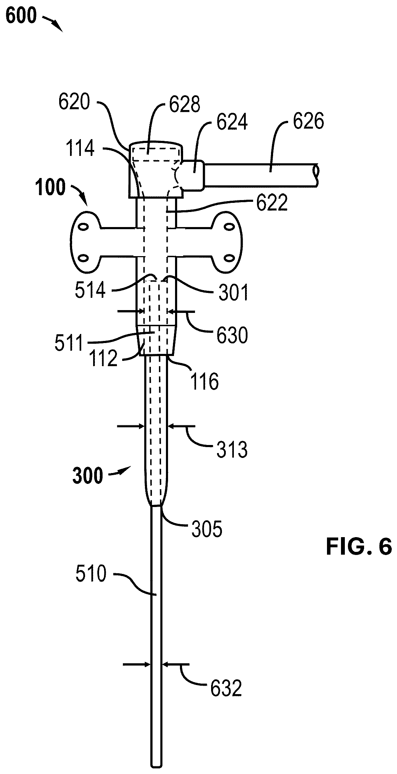

Referring to FIG. 6, in some embodiments of a system **600,** a hub **620** may be operably coupled to the second end **114** of the lumen. In some embodiments, the hub may be axially separated from the proximal end **514** of the tubular member by an intermediate portion **622** of the lumen. In this example, the proximal end **514** of the tubular member also may be the proximal end **301** of the sheath insert, but as will be understood, this is not necessary - the proximal end **514** and the proximal end **301** may be at different axial locations relative to each other. Further, in some embodiments, the proximal end **514** of the tubular member is also the proximal end **301** of the sheath insert are both coupled to the hub **620.**

The hub may comprise a hemostasis valve **628.** The hub may comprise a side port **624,** and a secondary tubular member **626** may be coupled to the side port.

As seen, the tubular member **510** may have an outer diameter **632** that is configured to match the diameter of the lumen **305** of the sheath insert. For example, the outer diameter **632** could have an interference fit with the lumen **305.** The outer diameter **313** of the sheath insert may then be configured to fit just within inner diameter **630** of lumen **116** of the suture pad **100.** In this way, the system can maintain hemostasis even if the tubular member has a much smaller outer diameter than the opening through which the system is advanced. As will be understood, in this example, another medical device (such as a blood pump) may be inserted through the hub **620,** through the lumen **116** of the suture pad and the lumen **305** of the sheath insert.

Various modifications may be made to the systems, methods, apparatus, mechanisms, techniques and portions thereof described herein with respect to the various figures, such modifications being contemplated as being within the scope of the invention. For example, while a specific order of steps or arrangement of functional elements is presented in the various embodiments described herein, various other orders/arrangements of steps or functional elements may be utilized within the context of the various embodiments. Further, while modifications to embodiments may be discussed individually, various embodiments may use multiple modifications contemporaneously or in sequence, compound modifications and the like.

Although various embodiments which incorporate the teachings of the present invention have been shown and described in detail herein, those skilled in the art can readily devise many other varied embodiments that still incorporate these teachings. Thus, while the foregoing is directed to various embodiments of the present invention, other and further embodiments of the invention may be devised without departing from the basic scope thereof. As such, the appropriate scope of the invention is to be determined of the claims.

The descriptions and drawings merely illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be only for illustrative purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or, unless otherwise indicated (*e.g.*, "or else" or "or in the alternative"). Also, the various embodiments described herein are not necessarily mutually exclusive, as some embodiments can be combined with one or more other embodiments to form new embodiments.

The numerous innovative teachings of the present application will be described with particular reference to the presently preferred exemplary embodiments. However, it should be understood that this class of embodiments provides only a few examples of the many advantageous uses of the innovative teachings herein. In general, statements made in the specification of the present application do not necessarily limit any of the various claimed inventions. Moreover, some statements may apply to some inventive features but not to others. Those skilled in the art and informed by the teachings herein will realize that the invention is also applicable to various other technical areas or embodiments.

## Claims

1. A suture pad (100) and sheath insert (300), comprising:
a central body (110) having an outer surface (111), a first end (112) and a second end (114), the central body (110) defining a lumen (116) extending from the first end (112) to the second end (114), the lumen (116) defining a central axis (118) of the suture pad and sheath insert; and
two wings (120), each wing extending radially outward from the outer surface of the central body (110), each wing defining a plurality of openings (126) extending from a first surface (122) of the wing to a second surface (124) of the wing (120) opposite the first surface (122);
wherein the two wings (122) are configured such that the suture pad (100) and sheath insert (300) is symmetrical around a first plane (128) parallel to and passing through the central axis (118), the first plane (128) passing between the first surface (122) and second surface (124) of each wing (120);
wherein the two wings (120) are configured such that the suture pad (100) and sheath insert (300) is symmetrical around a second plane (129) parallel to and passing through the central axis (118), the second plane (129) being perpendicular to the first plane (128).

2. The suture pad (100) and sheath insert (300) of claim 1, wherein an axial length (132) of the lumen (116) is the same as a minimum axial length (134) between a first end (113) of at least one of the two wings (120) and a second end (115) of the at least one of the two wings (120), or
wherein the axial length (132) of the lumen (116) is greater than the minimum axial length (134) between the first end (113) of at least one of the two wings (120) and the second end (115) of the at least one of the two wings (120).

3. The suture pad (100) and sheath insert (300) of claim 1 or 2, wherein an axial length (213) from the first end (112) of the central body (110) to a first end (113) of at least one of the two wings (120) is the same as the axial length (215) from the second end (114) of the central body (110) to a second end (115) of the at least one of the two wings (120),
or wherein the axial length (213) from the first end (112) of the central body (110) to the first end (113) of at least one of the two wings (120) is the greater than the axial length (215) from the second end (114) of the central body (110) to the second end (115) of the at least one of the two wings (120),
or wherein the axial length (213) from the first end (112) of the central body (110) to the first end (113) of at least one of the two wings (120) is the less than the axial length (215) from the second end (114) of the central body (110) to the second end (115) of the at least one of the two wings (120).

4. The suture pad (100) and sheath insert (300) of any one of the preceding claims, wherein the outer surface of the central body (110) at the first end (112) of the central body (110) is tapered (216) such that an outer diameter at the first end (112) is smaller than an outer diameter at a different portion of the central body (110).

5. The suture pad (100) and sheath insert (300) of any one of the preceding claims, wherein each wing (120) has a minimum axial length (134) between a first end (113) of the wing (120) and a second end (115) of the wing (120), and a maximum axial length (136) between a first end (113) of the wing (120) and a second end (115) of the wing (120), the minimum axial length (134) being different from the maximum axial length (136).

6. The suture pad (100) and sheath insert (300) of claim 5, wherein each wing (120) is configured such that the minimum axial length (134) is positioned at a point where the wing (120) contacts the central body (110).

7. The suture pad (100) and sheath insert (300) of any one of the preceding claims, wherein each wing (120) is configured such that the two wings (120) are symmetrical around a third plane (127) that is orthogonal to the central axis (118).

8. The suture pad (100) and sheath insert (300) of any one of the preceding claims, further comprising a sheath body (310), the sheath body (310) consisting of a cylindrical body having a proximal end (311), a distal end (302), and a sheath lumen (305) extending therethrough.

9. The suture pad (100) and sheath insert (300) of claim 8, wherein an outer surface of the sheath body (310) is coupled to an inner surface (117) of the lumen (116) of the central body (110).

10. A system (500, 600), comprising:
a suture pad (100) and sheath insert (300) of any one of the preceding claims; and
at least one other tubular member (510) disposed at least partially though the lumen (116, 305), an inner surface (117) of the lumen (116) of the suture pad (100) or a lumen (305) of a sheath body (310) forming an interference fit with the at least one other tubular member (510).

11. The system (500, 600) of claim 10, further comprising a hub (520) operably coupled to the at least one other tubular member (510).

12. The system (500, 600) of claim 10, further comprising a hub (620) operably coupled to the second end (114) of the lumen (116).

13. The system (500, 600) of claim 12, wherein the hub (620) comprises a hemostasis valve (628).

14. The system (500, 600) of claim 12 or 13, wherein the hub comprises a side port (624), wherein side port (624) is preferably coupled to a secondary tubular member (626).

15. The system of any one of the preceding claims 10 to 14, wherein the at least one other tubular member (510) is a repositioning sheath extending through the lumen (116).
